(19) Europäisches Patentamt
European Patent Office
Office européen des brevets

(11) Numéro de publication: **0 194 945**
**B1**

# (12) FASCICULE DE BREVET EUROPÉEN

(45) Date de publication du fascicule du brevet: **02.11.88**

(51) Int. Cl.⁴: **C 07 D 333/78**, C 07 D 409/04, A 61 K 31/38

(21) Numéro de dépôt: **86400526.9**

(22) Date de dépôt: **12.03.86**

(54) **Nouveaux dérivés de l'acide thiophène acétique, leur procédé de préparation, les intermédiaires obtenus, leur application à titre de médicaments et les compositions pharmaceutiques les renfermant.**

(30) Priorité: **12.03.85 FR 8503584**

(43) Date de publication de la demande: **17.09.86 Bulletin 86/38**

(45) Mention de la délivrance du brevet: **02.11.88 Bulletin 88/44**

(84) Etats contractants désignés: **BE CH DE GB IT LI LU NL**

(56) Documents cité:
**FR-A-2 346 348**

**CHEMICAL ABSTRACTS, vol. 95, no. 7, 17 août 1981, page 689, no. 61895d, Columbus, Ohio, US; P. STANETTY: "Thienospirans. Part 1. Spiro cyclohexane-cyclopenthatiophenes"**

(73) Titulaire: **ROUSSEL- UCLAF, 35, boulevard des Invalides, F-75007 Paris (FR)**

(72) Inventeur: **Clemence, François, 2, rue Turgot, F-75009 Paris (FR)**
Inventeur: **Le Martret, Odile, 42, avenue de Versailles, F-75016 Paris (FR)**
Inventeur: **Delevallee, Françoise, 48- 50, avenue de la Dame Blanche, F-94120 Fontenay- sous- bois (FR)**

(74) Mandataire: **Bourgouin, André, Département des Brevets ROUSSEL UCLAF B.P. no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 194 945 B1

LIBER, STOCKHOLM 1988

**Description**

La présente invention concerne de nouveaux dérivés de l'acide thiophène acétique, leur procédé de préparation, les intermédiaires obtenus, leur application à titre de médicaments et les compositions pharmaceutiques les renfermant.

D'autres dérivés de l'acide thiophène acétique sont déjà connus dans le brevet français 2 346 348 mais sont différents de ceux de la demande. En effet, d'une part ce ne sont pas des dérivés spirodécanes et d'autre part, ils sont différents au plan de leurs activités pharmacologiques.

L'invention a pour objet les produits de formule (I):

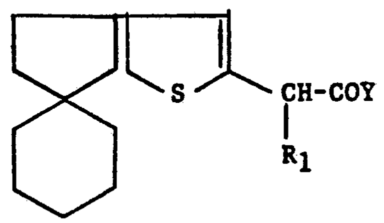

(I)

dans laquelle $R_1$ est un atome d'hydrogène ou un radical alcoyle renfermant de 1 à 5 atomes de carbone et Y représente soit un groupement $OR_2$ dans lequel $R_2$ est un atome d'hydrogène ou un radical alcoyle renfermant de 1 à 5 atomes de carbone ou un radical

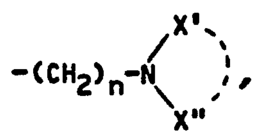

n étant un nombre entier pouvant varier de 2 à 5, X'et X'', identiques ou différents, représentent un atome d'hydrogène ou un radical alcoyle renfermant de 1 à 5 atomes de carbone ou X' et X'' forment avec l'atome d'azote auxquels ils sont liés un radical hétérocyclique, saturé ou non saturé, renfermant 5 ou 6 chaînons soit Y représente un groupement:

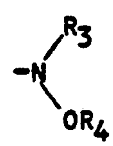

dans lequel $R_3$ et $R_4$ identiques ou différents représentent un atome d'hydrogène ou un radical alcoyle renfermant de 1 à 5 atomes de carbone, sous leurs formes racémique ou optiquement actives ainsi que leurs sels d'addition avec les bases et les acides.

Lorsque $R_1$, $R_2$, $R_3$ et $R_4$ représentent un radical alcoyle, il s'agit de préférence du radical méthyle, éthyle, n-propyle, n-butyle, n-pentyle, isopropyle, isobutyle ou tert.-butyle.

Le radical préféré pour $R_1$ est le radical méthyle.

n est de préférence 2 ou 3, X' et X'' sont identiques ou différents et représentent de préférence un atome d'hydrogène, un radical méthyle ou un radical éthyle. Lorsque X' et X'' forment avec l'atome d'azote auxquel ils sont liés un radical hétérocyclique, il s'agit de préférence d'un radical pipéridinyle, pyrrolidinyle, pipérazinyle, morpholinyle.

Parmi les sels d'addition avec les acides, on peut citer ceux formés avec les acides minéraux tels que les acides chlorhydrique, bromhydrique, sulfurique ou phosphorique ainsi que ceux formés avec les acides sulfoniques tels que les acides alcoyles ou arylsulfoniques, par exemple, l'acide méthanesulfonique ou paratoluènesulfonique.

Parmi les sels d'addition avec les bases, on peut citer ceux formés avec les métaux alcalins comme le sodium et le potasium et les amines, par exemple, la triméthylamine ou la diméthylamine.

L'invention a notamment pour objet l'acide 4',5'-dihydro '-méthyl spiro /cyclohexane-1,6'-/6H/-cyclopenta (b) thiophène/ 2'-acétique sous forme racémique ou optiquement actives ainsi que ses sels d'addition avec les bases.

L'invention a aussi pour objet un procédé de préparation des produits de formule (I), caractérisé en ce que l'on réduit le spiro /cyclohexane-1,6'-/6H/-cyclopenta (b) thiophène/-4'-(5'-H)-one de formule (A):

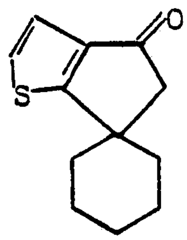

(A)

pour obtenir le spiro 4',5'-dihydro /cyclohexane-1,6'-/6H/-cyclopenta (b) thiophène/ de formule (B):

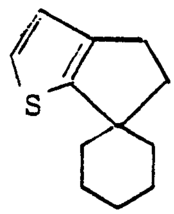

(B)

que l'on fait réagir en présence d'un acide de Lewis, avec un dérivé d'un acide de formule (II):

HOOC-COOR' (II)

R' étant un radical alcoyle renfermant de 1 à 5 atomes de carbone, pour obtenir un produit de formule (III):

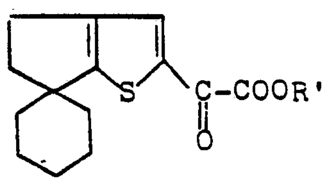

(III)

que l'on saponifie pour obtenir l'acide 4',5'-dihydro α'-oxo spiro /cyclohexane 1,6'-/6H/ cyclopenta (b) thiophène/ 2'-acétique de formule

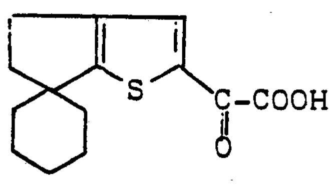

(C)

et que:

- soit l'on réduit la cétone de l'acide précité en méthylène pour obtenir un produit de formule (I) dans laquelle $R_1$ et $R_2$ représentent un atome d'hydrogène;
- soit l'on traite l'acide précité par un halogénure organomagnésien de formule $XMgR_1$, X étant un atome d'halogène et $R_1$ un radical alcoyle renfermant de 1 à 5 atomes de carbone, pour obtenir un sel de magnésien de formule (IV):

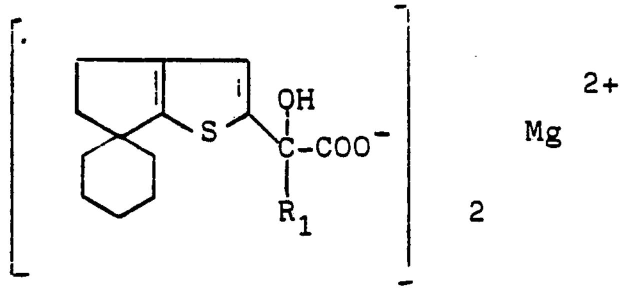

(IV)

que l'on réduit pour obtenir un produit de formule (I) dans laquelle $R_1$ a la signification donnée précédemment et Y représente le groupe $OR_2$ dans lequel $R_2$ est un atome d'hydrogène, et que si désiré, l'on estérifie les produits de formule (I) ainsi obtenus dans laquelle $R_2$ est un atome d'hydrogène en produit de formule (I) dans laquelle Y représente le groupe $OR_2$ dans lequel $R_2$ est un radical alcoyle renfermant de 1 à 5 atomes de carbone ou un radical:

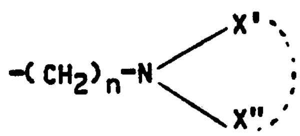

et que le cas échéant on traite les produits de formule (I) ainsi obtenus par une hydroxylamine de formule:

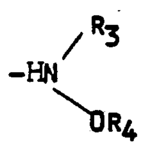

dans laquelle $R_3$ et $R_4$ ont les significations indiquées précédemment pour obtenir les produits de formule (I) dans laquelle Y représente un groupement:

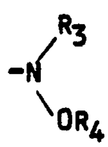

produits de formule (I) que l'on peut, le cas échéant, dédoubler par des méthodes usuelles ou transformer en sels par action d'un acide ou d'une base.

L'invention a également pour objet une variante du procédé de préparation des produits de formule (I) caractérisé en ce que l'on fait réagir le produit de formule (B) en présence d'un acide de Lewis avec un dérivé bromé d'un acide de formule (II'):

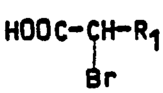

(II')

dans lequel $R_1$ a la signification indiquée précédemment pour obtenir un produit de formule (V):

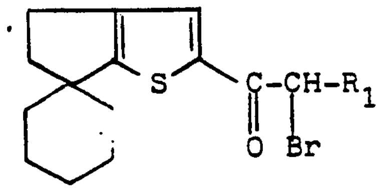

(V)

dans laquelle $R_1$ a la signification précédente, produit de formule (V) que l'on transforme en milieu acide par action d'un diol approprié en acétal cyclique de formule VI:

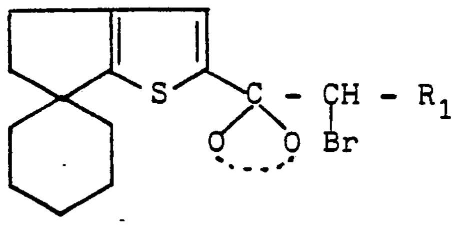

(VI)

dans laquelle $R_1$ a la signification précédente et le trait pointillé représente le reste du diol utilisé que l'on transforme par chauffage au sein d'un solvant polaire en ester de formule VII:

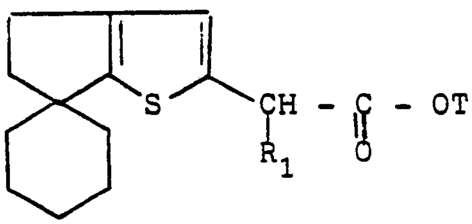

(VII)

dans laquelle $R_1$ a la signification précédente et T représente un reste alcoolique qui est fonction du diol et du solvant polaire utilisés, produit de formule VII que l'on saponifie pour obtenir le produit de formule (I) dans lequel Y représente le goupe $OR_2$ dans lequel $R_2$ est un atome d'hydrogène, et $R_1$ a la signification indiquée précédemment et que le cas échéant, l'on traite les produits de formule (I) ainsi obtenus, comme indiqué dans le procédé décrit ci-dessus.

Dans un mode de réalisation préféré du procédé de l'invention:

- Le dérivé de l'acide de formule (II) est le chlorure d'acide; on peut également utiliser d'autres dérivés de cet acide tels que l'anhydride d'acide ou un autre halogénure.

Dans l'acide de formule (II), R' est un radical méthyle ou éthyle. La réaction avec cet acide s'effectue en présence d'un acide de Lewis qui est le chlorure d'aluminium; d'autres réactifs du même type peuvent, bien entendu, être utilisés, par exemple, le chlorure stannique, le chlorure de zinc, le trifluorure de bore, l'acide phosphorique.

- La saponification du produit de formule (III) s'effectue dans des conditions usuelles.
- La réduction du carbonyle en méthylène du produit de formule

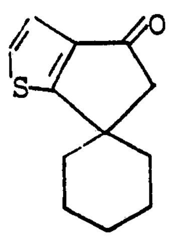

(A)

et de formule

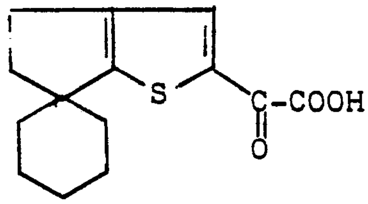

(C)

s'effectue par une réaction de Wolff-Kishner en présence d'hydrate d'hydrazine d'éthylène-glycol et de potasse.

- L'halogénure organomagnésien est l'iodure de méthyl magnésium; il agit avantageusement en présence d'un mélange éther éthylique-tétrahydrofurane.

- La réduction du sel de magnésien de formule (IV) s'effectue par du chlorure stanneux. D'autres procédés de réduction, tels que l'hydrogénation catalytique peuvent être utilisés.

- L'estérification des acides de formule (I) est réalisée de façon usuelle, par exemple, par action d'un alcool ou d'un aminoalcool ou d'un halogénure d'alcoyle ou un halogénure d'aminoalcoyle ou par exemple, par action du diazométhane pour la méthylation.

- La préparation d'acides hydroxamiques de formule (I) dans laquelle Y représente le groupement:

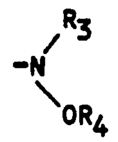

est effectuée par action du chlorhydrate d'hydroxylamine ou l'un de ses dérivés sur un produit de formule (I) dans laquelle Y représente un groupement $OR_2$ dans lequel $R_2$ représente un atome d'hydrogène au sein d'un solvant organique tel que le chlorure de méthylène après activation de la fonction carboxyle en présence de préférence de carbonyldiimidazole.

Dans un mode de réalisation préféré de la variante du procédé de l'invention:

- le dérivé bromé d'acide de formule (II') est de préférence un chlorure d'acide α-bromé.

- la réaction du produit de formule (B) avec le dérivé de formule (II') s'effectue dans des conditions identiques à celles indiquées pour la réaction du produit de formule (B) avec le dérivé de formule (II):

- la transformation de la cétone de formule (V) en acétalcyclique de formule (VI) s'effectue par action du 2,2-diméthyl 1,3-propane diol au sein d'un solvant organique tel que le toluène en présence d'un acide tel que l'acide paratoluène sulfonique, en chauffant au reflux du mélange réactionnel,

- la transformation de l'acétal de formule (VI) en ester de formule (VII) s'effectue par chauffage dans un solvant polaire à une température comprise entre 50° C et la température d'ébullition du solvant; le solvant polaire est l'éthane diol éventuellement substitué et la réaction est catalysée par un acétate de métal alcalin tel que l'acétate de potassium;

- la valeur de T dans le produit de formule (VII) est fonction du diol et du solvant polaire utilisés, c'est-à-dire que le produit de formule (VII) peut être le cas échéant un ester ou un mélange d'esters.

- la saponification du produit de formule (VII) s'effectue dans les conditions usuelles.

Les composés de formule (I) tels que définis ci-dessus, ainsi que leurs sels d'addition avec les acides et les bases présentent d'intéressantes propriétés pharmacologiques. Ils présentent en particulier, une bonne activité analgésique et anti-inflammatoire et notamment un pouvoir anti-arthritique important. Ce sont aussi des inhibiteurs actifs de la 5-lipoxygénase et de la cyclo-oxygénase.

Ces propriétés justifient leur application en thérapeutique et l'invention a également pour objet à titre de médicaments, les produits tels que définis par la formule (I) ci-dessus, ainsi que les sels d'addition avec les acides et les bases pharmaceutiquement acceptables desdits produits de formule (I).

L'invention a plus particulièrement pour objet, à titre de médicaments l'acide 4',5'-dihydro-α'-méthyl-spiro /cyclohexane-1,6'-/6H/-cyclopenta (b) thiophène/ 2'-acétique sous forme racémique ou optiquement actives ainsi que ses sels d'addition avec les bases pharmaceutiquement acceptables.

Les médicaments, objets de l'invention, peuvent être préconisés dans le traitement des maladies inflammatoires dégénératives telles que l'ostéoarthrose, des collagénoses diverses (tendinites, etc...), des maladies rhumatismales (la polyarthrite rhumatoïde, spondylarthrite ankylosante), ainsi que dans le traitement d'autres maladies de nature auto-immune telles que le lupus érythémateux disséminé, les glomérulonéphrites, la sclérose en plaques, etc...

Les médicaments objets de l'invention peuvent également être utilisés dans le traitement des algies musculaires, articulaires ou nerveuses, les douleurs dentaires, les migraines, le zona et également, à titre de traitement complémentaire dans les états infectieux et fébriles. Ils peuvent aussi être utilisés pour traiter le psoriasis, l'asthme et certains désordres cardiovasculaires tels que l'infarctus du myocarde.

L'invention s'étend aux compositions pharmaceutiques renfermant comme principe actif, les médicaments définis ci-dessus.

Ces compositions pharmaceutiques peuvent être administrées par voie buccale, par voie rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration de l'affection traitée et du sujet en cause.

Par exemple, chez l'adulte, elle peut varier entre 20 mg et 2 g de principe actif par jour, par voie orale.

Les produits de formules (III), (IV), (V) et (VI) tels que définis ci-dessus et notamment:

- le spiro 4',5'-dihydro /cyclohexane-1,6'- /6H/-cyclopenta (b) thiophène et
- l'acide 4',5'-dihydro-α'-oxo spiro /cyclohexane 1,6'-/6H/-cyclopenta (b) thiophène/ 2'-acétique

sont des produits chimiques nouveaux; l'invention a donc pour objet ces produits à titre de produits industriels nouveaux, notamment à titre de produits intermédiaires nécessaires à la mise en oeuvre du procédé de l'invention.

Les exemples donnés ci-après illustrent l'invention sans toutefois la limiter.

EXEMPLE 1

Acide 4',5'-dihydro-α'-méthyl-spiro /cyclohexane-1,6'-/6H/-cyclopenta (b) thiophène/ 2'-acétique.

Stade A: Spiro 4',5'-dihydro /cyclohexane 1,6'-/6H/-cyclopenta (b) thiophène/.

On agite à température ambiante 17 g de spiro /cyclohexane-1,6'-/6H/-cyclopenta (b) thiophène/-4'-(5'-H)-one (préparé selon le procédé décrit par Peter Stanelty, J. Chem. Res (8), 1981, 99) et 55 $cm^3$ d'éthylène glycol, ajoute goutte à goutte 19,9 $cm^3$ d'une solution de potasse à 700 g/l. La température s'élève à +45° C. On ajoute 30,7 $cm^3$ d'hydrate d'hydrazine goutte à goutte, chauffe 1 heure au reflux à +125°C la solution, et, distille à pression ambiante l'excès d'eau. Après distillation, la température s'élève à +195°C, on laisse alors au reflux pendant 3 heures à cette température. On verse la solution obtenue dans 150 $cm^3$ d'eau puis ajoute 150 $cm^3$ d'éther éthylique. On décante la phase organique puis extrait à l'éther. On récupère les phases organiques, les lave à l'eau jusqu'à neutralité, sèche, purifie avec du charbon actif, filtre et concentre sous pression réduite.

On purifie l'huile ainsi obtenue par chromatographie sur colonne haute pression en éluant avec de l'éther de pétrole (Eb: 60 - 80° C). On obtient le produit attendu sous forme d'une huile incolore. (Rf = 0,6).

Analyse: $C_{12}H_{16}S$ = 192,3

| | | | |
|---|---|---|---|
| Calculé: | C% 74,95 | H% 8,40 | S% 16,65 |
| Trouvé: | 75,2 | 8,6 | 16,6 |

Stade B: 4',5'-dihydro α'-oxo spiro /cyclohexane 1,6'-/6H/-cyclopenta (b) thiophène/ 2'-acétate d'éthyle.

On refroidit à 0°C +5°C le mélange renfermant 80 $cm^3$ de 1,2-dichloroéthane et 9 g de chlorure d'aluminium puis introduit en 7 minutes à +5°C/+6°C, la solution renfermant 6,9 $cm^3$ de chloro oxo acétate d'éthyle et 10 $cm^3$ de 1,2-dichloroéthane. On agite 15 minutes à 0°C/+5°C, refroidit à - 5°C et introduit en 30 minutes 10,2 g de produit obtenu au stade A en solution dans 20 $cm^3$ de 1,2-dichloroéthane. On agite à 0°/+5°C pendant 4 heures, laisse revenir à +20°C et agite 16 heures à cette température. On verse la solution sous agitation dans le mélange eau + glace (265 $cm^3$) et acide chlorhydrique 22°Be (53 $cm^3$), rince par 100 $cm^3$ de chlorure de méthylène, agite 1 heure, extrait au chlorure de méthylène, lave à l'eau, distille à sec sous pression réduite. On obtient 14,5 g de produit attendu présentant un Rf de 0,49 en chromatographie sur silice en éluant avec du chlorure de méthylène.

Stade C: Acide 4',5'-dihydro-α'-oxo spiro /cyclohexane-1,6'-/6H/-cyclopenta (b) thiophène/ 2'-acétique.

On introduit à 18 - 20°C, goutte à goutte, sous argon 26,7 $cm^3$ de soude 2N dans une solution renfermant 14,2 g de produit obtenu au stade B, 18 $cm^3$ d'éthanol et 36 $cm^3$ d'eau. On agite 2 heures, ajoute 180 $cm^3$ d'eau, filtre, rince à l'eau, lave par 2 fois 100 $cm^3$ d'éther, éthylique, réextrait les lavages éthérés par 50 $cm^3$ d'eau. On acidifie à pH 1 par de l'acide chlorhydrique 22°Bé en présence de 150 $cm^3$ d'acétate d'éthyle, décante, réextrait par 2 fois 100 $cm^3$ d'acétate d'éthyle, lave avec une solution aqueuse d'hydroxyde de sodium (70 g/l), sèche les phases organiques, filtre sur charbon actif et distille à sec sous pression réduite. La résine obtenue est empâtée dans l'éther de pétrole (Eb = 60 - 80°C), on essore, rince à l'éther de pétrole (Eb = 60 - 80°C), sèche et obtient 10,9 g de produit attendu. Le produit présente un Rf de 0,43 en chromatographie sur silice (éluant: acétate d'éthyle-éthanol-eau (7-2-1)).

Stade D: Sel de magnésium de l'acide 4',5'-dihydro- '-hydroxy-α'-méthyl spiro /cyclohexane-1,6'-/6H/-cyclopenta (b) thiophène/ 2'-acétique.

On introduit sous argon à +6°/+8°C, 42 $cm^3$ d'iodure de méthyl magnésium dans l'éther éthylique dans une solution renfermant 7,97 g de produit obtenu au stade B dans 126 $cm^3$ de tétrahydrofuranne. On agite 45 minutes à 0°/+5°C puis 2 heures à 20°/25°C. On introduit lentement à +10°/+15°C une solution renfermant 18 g de chlorure d'ammonium dans 180 $cm^3$ d'eau. On ramène à +20°C, distille sous pression réduite, essore le produit cristallisé, rince avec de l'eau puis avec de l'éther éthylique, sèche et obtient 8,65 g de produit attendu (point de fusion à l'acide correspondant F - 160°C).

Analyse: $C_{30}H_{38}O_6S_2$ Mg = 583,073

| | | | |
|---|---|---|---|
| Calculé: | C% 61,8 | H% 6,57 | Mg% 4,7 |
| Trouvé: | 58,5 | 6,8 | 4,7 |

Stade E: Acide 4',5'-dihydro-α'-méthyl spiro /cyclohexane-1,6'-/6H/-cyclopenta (b) thiophène/ 2'-acétique.

On introduit à +18°/+21°C, par petites fractions, 8,65 g de produit obtenu au stade D dans un mélange contenant 10 g de chlorure stanneux, 15 $cm^3$ d'acide chlorhydrique 22°Bé, et 28,5 $cm^3$ d'acide acétique. On agite 5 heures à 20°/25°C, obtient des cristaux, essore, rince à l'acide acétique, puis à l'eau et sèche sous pression réduite à +20°/25°C. On dissout 5,89 g du produit brut obtenu dans 30 $cm^3$ d'acide acétique à +20°C, filtre sur charbon actif, rince à l'acide acétique. On ajoute au filtrat à 20°C, 19 $cm^3$ d'eau goutte à goutte en amorçant la cristallisation. On essore le produit obtenu, rince avec une solution aqueuse d'acide acétique (30 %), sèche sous pression réduite à 20°C. On obtient 5,21 g de produit attendu que l'on repurifie comme indiqué précédemment et obtient 4,85 g de produit attendu fondant à +95°C.

Analyse: $C_{15}H_{20}O_2S$ = 264,389

| | | | |
|---|---|---|---|
| Calculé: | C% 68,14 | H% 7,62 | S% 12,13 |
| Trouvé: | 68,3 | 7,8 | 12,0 |

EXEMPLE 2

Oxalate de 4',5'-dihydro-α'-méthyl spiro /cyclohexane 1,6'-/6H/-cyclopenta (b) thiophène/ 2'-acétate de 2-(diméthylamino) éthyle.

On agite sous argon à 20° - 22°C durant 22 heures une suspension contenant 132 mg de produit de l'exemple 1,144 mg de chlorhydrate de 2-chloro-N,N-diméthyl aminoéthane, 520 mg de carbonate de potassium, 2 $cm^3$ de diméthylformamide. On verse sur 50 $cm^3$ d'eau, filtre sur silice, rince à l'eau, dissout le produit dans le chlorure de méthylène, filtre, sèche et concentre à sec sous pression réduite. On reprend 55 mg d'huile incolore obtenue par 0,5 $cm^3$ d'isopropanol et ajoute 25 mg d'acide oxalique bihydraté. On essore l'oxalate cristallisé, rince à l'isopropanol puis à l'éther éthylique et obtient 43 mg de produit attendu fondant à 144°C.

Analyse:

| | | | | |
|---|---|---|---|---|
| Calculé: | C% 59,27 | H% 7,34 | N% 3,29 | S% 7,53 |
| Trouvé: | 59,1 | 7,5 | 3,2 | 7,5 |

EXEMPLE 3

4',5'-dihydro-α'-méthyl spiro /cyclohexane-1,6'-/6H/-cyclopenta (b) thiophène/-2'-acétate de méthyle.

On introduit en 5 minutes à +15°/+20°C en refroidissant par un mélange eau + glace, 20 $cm^3$ d'une solution 0,4M de diazométhane dans le chlorure de méthylène dans un mélange renfermant 1,5 g de produit de l'exemple 1 dans 5 $cm^3$ de chlorure de méthylène. On extrait au chlorure de méthylène, lave à l'eau, sèche, agite 5 minutes en présence de 2 g de silice, filtre, concentre à sec sous pression réduite et obtient 1,579 g de produit attendu sous forme d'une huile incolore présentant un Rf de 0,73 en chromatographie sur silice (éluant: chlorure de méthylène-acétate d'éthyle 1 - 1).

EXEMPLE 4

4',5'-dihydro α,N-diméthyl N-hydroxy spiro /cyclohexane-1,6'/6H/-cyclopenta (b) thiophène/2'-acétamide et son sel de sodium.

On agite à température ambiante pendant 30 minutes sous atmosphère inerte 5,3 g de produit préparé comme à l'exemple 1 et 5,2 g de carbonyl diimidazole dans 55 $cm^3$ de chlorure de méthylène. On ajoute 3,5 g de chlorhydrate de N-méthyl hydroxylamine et maintient sous agitation pendant 72 heures.

On filtre la solution, lave le filtrat avec 100 $cm^3$ d'acide chlorhydrique N, puis à l'eau, sèche et concentre à

sec.

On obtient 6,1 g de produit brut que l'on chromatographie sur silice (éluant chlorure de méthylène - acétate d'éthyle 8 - 2).

On recueille 3,3 g de produit pur. Rf. = 0,3

Préparation du sel de sodium

On dissout 2,7 g du produit obtenu précédemment dans 13,5 $cm^3$ d'éthanol 100° puis ajoute une solution de 6,75 $cm^3$ d'hydroxyde de sodium à 32 % dans 27 $cm^3$ d'eau.

On glace pendant 15 minutes, essore et lave les cristaux à l'eau glacée, sèche sous pression réduite à température ambiante et recueille 1,73 g de sel de sodium du produit attendu. F = 120°C (avec décomposition).

Analyse: $C_{16}H_{23}NO_2S, 3H_2O,Na$ = 369,459

| | | | | |
|---|---|---|---|---|
| Calculé: | C % 52,01 | H% 7,64 | S% 8,68 | N% 3,79 |
| Trouvé: | 52,2 | 7,5 | 8,4 | 3,5 |

EXEMPLE 5

4',5'-dihydro N-hydroxy α-méthyl spiro/cyclohexane-1,6' (6H)-cyclopenta (b) thiophène/2'-acétamide.

On opère comme à l'exemple 4 en utilisant au départ 4 g de produit obtenu à l'exemple 1 et en employant à la place du chlorhydrate de N-méthyl hydroxylamine, 2,2 g de chlorhydrate d'hydroxylamine.

On maintient le mélange sous agitation 5 heures à température ambiante, filtre la suspension, lave le filtrat avec 100 $cm^3$ d'acide chlorhydrique N puis à l'eau, sèche, concentre à sec sous pression réduite, récupère 4,2 g de produit brut qui cristallise. On l'empâte dans 30 $cm^3$ d'hexane, essore, lave à l'hexane et sèche sous pression réduite. Après recristallisation dans l'acétate d'éthyle, on recueille 2,15 g de produit attendu pur. F = 135°C.

Analyse: $C_{15}H_{21}NO_2S$ = 279,404

| | | | | |
|---|---|---|---|---|
| Calculé: | C % 64,48 | H% 7,58 | S% 11,48 | N% 5,01 |
| Trouvé: | 64,4 | 7,7 | 11,3 | 5,0 |

EXEMPLE 6

4',5'-dihydro N-hydroxy α-méthyl N-(1-méthyléthyl) spiro /cyclohexane 1,6'(6H)-cyclopenta (b) thiophène/2'-acétamide.

On opère comme à l'exemple 4 en utilisant au départ 4 g de produit obtenu à l'exemple 1 et en employant à la place du chlorhydrate de N-méthyl hydroxylamine 3,55 g de chlorhydrate de N-isopropyl hydroxylamine et en laissant réagir une heure 30 minutes.

Après chromatographie sur silice du produit brut (éluant chlorure de méthylène) on obtient 4,4 g de produit attendu pur, sous forme d'huile.

Analyse: $C_{18}H_{27}NO_2S$ = 321,486

| | | | | |
|---|---|---|---|---|
| Calculé: | C % 67,25 | H % 8,47 | S % 9,97 | N % 4,36 |
| Trouvé: | 67,2 | 8,5 | 9,9 | 4,4 |

EXEMPLE 7

4',5'-dihydro α,N-diméthyl N-méthoxy spiro /cyclohexane 1,6' (6H) cyclopenta (b) thiophène/2'-acétamide.

On opère comme à l'exemple 4 en utilisant au départ 4 g du produit obtenu à l'exemple 1 et en employant à la place du chlorhydrate de N-méthyl hydroxylamine 3,1 g de chlorhydrate de méthoxy méthylamine en laissant réagir 20 heures.

On recueille après la chromatographie sur silice (éluant: chlorure de méthylène - acétate d'éthyle 8 - 2) 3,65 g de produit attendu.

Rf = 0,6.

Analyse: $C_{17}H_{25}NO_2S$ = 307,459

| | | | | |
|---|---|---|---|---|
| Calculé: | C % 66,41 | H % 8,20 | S % 10,43 | N % 4,55 |
| Trouvé: | 66,5 | 8,4 | 10,1 | 4,4. |

EXEMPLE 8

Acide 4',5'-dihydro spiro /cyclohexane 1,6' (6H)-cyclopenta (b) thiophène/2'-acétique.

On dissout à 100°C 4,25 g de potasse dans 36 $cm^3$ de diéthylène glycol, refroidit à 40°C et ajoute 5 g d'acide préparé comme au stade C de l'exemple 1 dans 3,7 $cm^3$ de monohydrate d'hydrazine.

On chauffe progressivement jusqu'à 200°C en éliminant l'eau par distillation et maintient sous agitation à 200°C pendant une heure 30 minutes. On laisse revenir à température ambiante, verse le milieu réactionnel dans 100 $cm^3$ d'eau, lave la phase aqueuse par 200 $cm^3$ d'acétate d'éthyle, ajoute 15 $cm^3$ d'acide chlorhydrique concentré et extrait à l'acétate d'éthyle.

On réunit les phases organiques, les lave à l'eau, les sèche, ajoute du charbon actif, filtre, concentre à sec le filtrat sous pression réduite et obtient 5,29 g de produit brut qui cristallise et que l'on empâte dans l'hexane. On essore, sèche le produit sous pression réduite et obtient 3,26 g de produit attendu que l'on recristallise dans l'hexane. F = 92°C

Analyse: $C_{14}H_{18}O_2S$ = 250,362

| | | | |
|---|---|---|---|
| Calculé: | C % 67,16 | H % 7,25 | S % 12,81 |
| Trouvé: | 67,2 | 7,3 | 12,5. |

EXEMPLE 9

Acide 4',5'-dihydro α-méthyl spiro /cyclohexane 1,6 (6H)-cyclopenta (b) thiophène/2-acétique.

Stade A: 2-bromo-1-/4',5'-dihydro spiro/cyclohexane 1,6 (6H) cyclopenta (b) thiophen/2'-yl/1-propanone.

On dissout en partie à chaud 8,7 g de chlorure d'aluminium dans 80 $cm^3$ de dichloroéthane, refroidit à 0°C, ajoute en 5 minutes à cette température une solution comprenant 10,28 g de chlorure d'α-bromo propionyle dans 30 $cm^3$ de dichloroéthane, puis en 15 minutes, en maintenant la température à 0°/5°C une solution comprenant 9,61 g de spiro /cyclohexane 1,6'/6H/-cyclopenta (b) thiophène/4'(5'H)-one dans 20 $cm^3$ de dichloroéthane. On laisse revenir à température ambiante, puis chauffe au reflux pendant une heure, refroidit, ajoute 200 $cm^3$ d'acide chlorhydrique N, lave à l'eau, ajoute du charbon actif, filtre, sèche, concentre et recueille 16,52 g de produit brut que l'on reprend dans 50 $cm^3$ d'hexane, chauffe au reflux, laisse refroidir et essore le produit cristallisé.

On obtient 11,77 g de produit attendu. F < 50°C.

Stade B: 2-(1-bromoéthyl)2-/4',5'-dihydrospiro /cyclohexane-1,6 (6H)-cyclopenta (b) thiophène/2'-yl/ 1,3-dioxane.

On chauffe au reflux pendant 5 heures 30 minutes 5 g de produit obtenu au stade précédent 3,12 g de 2,2-diméthyl 1,3-propanediol, 0,28 g d'acide paratoluènesulfonique dans 20 $cm^3$ de toluène en éliminant l'eau formée au cours de la réaction.

On refroidit le milieu réactionnel, élimine par filtration l'insoluble, lave la phase organique avec une solution aqueuse de carbonate de sodium à 10 % puis à l'eau, sèche, concentre à sec et obtient 5,95 g de produit attendu utilisé tel quel pour le stade suivant.

Stade C: Acide 4',5'-dihydro α-méthyl spiro /cyclohexane 1,6'(6H)-cyclopenta (b) thiophène/2-acétique.

On chauffe 5 heures à 125°C, 2,06 g de produit obtenu au stade précédent et 0,5 g d'acétate de potassium dans 25 $cm^3$ d'éthanediol 1 - 2. On refroidit, verse sur 50 $cm^3$ d'eau et extrait à l'acétate d'éthyle, sèche, concentre à sec et recueille 1,6 g d'ester.

On ajoute 5 $cm^3$ de méthanol et 1 $cm^3$ de lessive de soude au produit obtenu ci-dessus, chauffe au reflux pendant une heure 30 minutes, chasse le méthanol, dilue avec 10 $cm^3$ d'eau et extrait à la méthyléthylcétone.

La phase aqueuse est acidifiée à pH 1 avec de l'acide chlorhydrique concentré et on extrait à nouveau, on réunit les phases organiques, les lave à l'eau, les sèche et les concentre à sec. On obtient 1,15 g de produit attendu, identique au produit obtenu à l'exemple 1.

EXEMPLE 10

Compositions pharmaceutiques.

On a préparé des comprimés répondant à la formule suivante:

| | |
|---|---|
| Produit de l'exemple 1 | 50 mg |
| Excipient q.s. pour un comprimé terminé à | 350 mg. |

(Détail de l'excipient: lactose, talc, amidon, stéarate de magnésium).

**ETUDE PHARMACOLOGIQUE**

1. Activité anti-inflammatoire: arthrite à l'adjuvant (traitement préventif)

L'injection d'adjuvant de type Freund dans une patte postérieure provoque, chez le rat, l'apparition rapide d'une lésion inflammatoire primaire dans cette patte, puis, après un temps de latence de 13 à 15 jours, le déclenchement d'une arthrite secondaire affectant notamment l'autre patte postérieure. Le test est pratiqué sur des rats mâles âgés de 40 à 50 jours, qui reçoivent en injection intraplantaire, 0,1 ml d'adjuvant de type Freund (suspension dans l'huile de vaseline de 6 mg par ml de mycobacterium butyricum tués).

Les animaux reçoivent le produit étudié, par voie orale, du jour 0 (jour de l'injection de l'adjuvant) jusqu'à la veille du sacrifice, pratiqué le jour 17. Des animaux témoins arthritiques, des animaux témoins normaux ne reçoivent que le véhicule. Les critères d'appréciation de l'activité des substances étudiées sont les augmentations de volume des pattes postérieures injectées (inflammation primaire et secondaire) et non injectées (inflammation secondaire) par rapport au volume moyen des pattes correspondantes de témoins arthritiques.

On détermine la $DA_{50}$, c'est-à-dire la dose qui diminue de 50 % les augmentations de volume des pattes postérieures des animaux traités par rapport aux animaux témoins.

La $DA_{50}$ trouvée a été de 8 mg/kg pour le produit de l'exemple 1.

2. Inhibition de la biosynthèse des prostaglandines in vitro

L'acide arachidonique est converti en prostaglandines (PGs) de la série 2 par une cyclo-oxygénase contenue dans une préparation microsomale de vésicules séminales de taureau effectuée selon la méthode de Tagekuchi et al. (1).

Le précurseur, à la concentration de 15 $\times 10^{-6}$M, est incubé en présence d'une concentration fixe en protéines de la préparation de vésicules séminales et du produit à tester pendant 30 minutes à 37°C. Après blocage de la réaction par immersion dans l'eau bouillante pendant une minute puis centrifugation, les prostaglandines sont dosées par radio-immunoessai. Cette technique, inspirée de celle de Dray et al. (2) permet d'évaluer spécifiquement les $PGE_2$ et $PGF_2\alpha$. L'activité inhibitrice des produits ($CI_{50}$) est calculée sur la somme de ces deux prostaglandines.

1) TAGEKUCHI, C., KUHNO E. and SIH, C.J.
Mechanism of prostaglandin biosynthesis. I characterisation and essay of bovine prostaglandin synthetase.
Biochemistry, 1971, 10, 2372.
2) DRAY F., CHARBONNEL B. and MACLOUF J.
Radioimmunoassay of prostaglandins F, $E_1$ and $E_2$ in human plasma.
European J. Clin. Invest., 1975, 5, 311.

Résultats:

Produit de l'exemple 1: $CI_{50} = 2 \times 10^{-5}$M.

3) Dosage de la 5-lipoxygénase de neutrophiles de rat

Les neutrophiles péritonéaux ($5 \times 10^{6}$ cellules/ml) obtenus après injection intrapéritonéale de caséinate de sodium à 12 % sont préincubés à 37°C pendant cinq minutes en présence des produits à tester ou du solvant utilisé. L'incubation a lieu dans les mêmes conditions et en présence du ionophore A 23187 (10 μM) et du chlorure de calcium (5 mM). Après cinq minutes, les incubats sont portés en glace et centrifugés à 3000 g. Le leucotriène $B_4$ est dosé dans le surnageant par radioimmunoessai.

Résultats exprimés en $CI_{50}$

| | 5-lipoxygénase | cyclooxygénase |
|---|---|---|
| Produit de l'exemple 1 | $1 \times 10^{-5}$M | $2 \times 10^{-5}$M |
| Produit de l'exemple 4 | $0{,}5 \times 10^{-6}$M | $2 \times 10^{-4}$M |

**Revendications**

1. Les produits de formule (I):

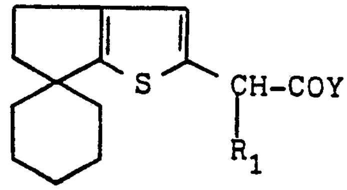

(I)

dans laquelle $R_1$ est un atome d'hydrogène ou un radical alcoyle renfermant de 1 à 5 atomes de carbone et Y représente soit un groupement $OR_2$ dans lequel $R_2$ est un atome d'hydrogène ou un radical alcoyle renfermant de 1 à 5 atomes de carbone ou un radical:

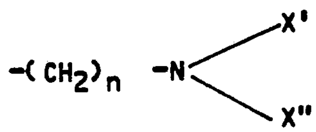

n étant un nombre entier pouvant varier de 2 à 5, X' et X'' identiques ou différents représentent un atome d'hydrogène ou un radical alcoyle renfermant de 1 à 5 atomes de carbone ou X' et X'' forment avec l'atome d'azote auxquels ils sont liés un radical hétérocyclique, saturé ou non saturé, renfermant 5 ou 6 chaînons soit Y représente un groupement:

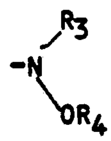

dans lequel $R_3$ et $R_4$ identiques ou différents représentent un atome d'hydrogène ou un radical alcoyle renfermant de 1 à 5 atomes de cabone, sous leurs formes racémique ou optiquement actives ainsi que leurs sels d'addition avec les bases et les acides.

2. L'acide 4',5'-dihydro α'-méthyl spiro /cyclohexane-1,6'/6H/-cyclopenta (b) thiophène/2'-acétique sous forme racémique ou optiquement actives ainsi que ses sels d'addition avec les bases.

3. Procédé de préparation des produits de formule (I), caractérisé en ce que l'on réduit le spiro /cyclohexane-1,6'/6H/-cyclopenta (b) thiophène/-4'-(5'-H)-one de formule (A):

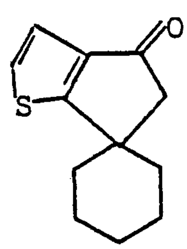

(A)

pour obtenir le spiro 4',5'-dihydro /cyclohexane-1,6'/6H/-cyclopenta (b) thiophène/ de formule (B):

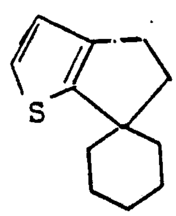

(B)

que l'on fait réagir en présence d'un acide de Lewis, avec un dérivé d'un acide de formule (II):

HOOC-COOR' (II)

R' étant un radical alcoyle renfermant de 1 à 5 atomes de carbone, pour obtenir un produit de formule (III):

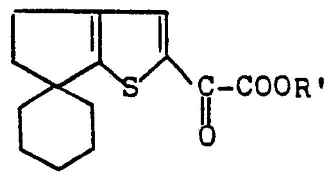

(III)

que l'on saponifie pour obtenir l'acide 4',5'-dihydro α'-oxo spiro /cyclohexane 1,6'/6H/-cyclopenta (b) thiophène/2'-acétique et que

- <u>soit</u> l'on réduit la cétone de l'acide précité en méthylène pour obtenir un produit de formule (I) dans laquelle $R_1$ et $R_2$ représentent un atome d'hydrogène;

- <u>soit</u> l'on traite l'acide précité par un halogénure organomagnésien de formule $XMgR_1$, X étant un atome d'halogène et $R_1$ un radical alcoyle renfermant de 1 à 5 atomes de carbone, pour obtenir un sel de magnésien de formule (IV):

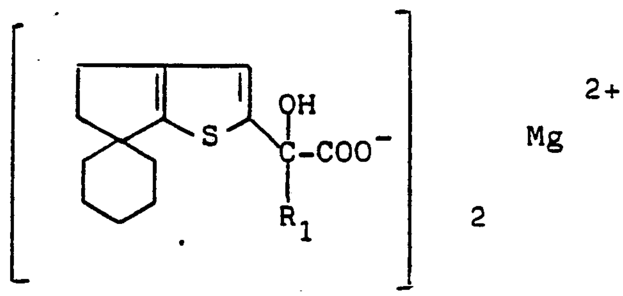

(IV)

que l'on réduit pour obtenir un produit de formule (I) dans laquelle $R_1$ a la signification donnée précédemment et Y représente le groupe $OR_2$ dans lequel $R_2$ est un atome d'hydrogène et que si désiré, l'on estérifie les produits de formule (I) ainsi obtenus dans laquelle $R_2$ est un atome d'hydrogène en produit de formule (I) dans laquelle Y représente le groupe $OR_2$ dans lequel $R_2$ est un radical alcoyle renfermant de 1 à 5 atomes de carbone ou un radical:

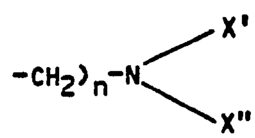

et que le cas échéant on traite les produits de formule (I) ainsi obtenus par une hydroxylamine de formule:

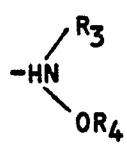

dans laquelle $R_3$ et $R_4$ ont les significations indiquées précédemment pour obtenir les produits de formule (I) dans laquelle Y représente un groupement:

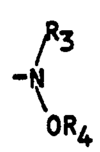

produits de formule (I) que l'on peut, le cas échéant, dédoubler par des méthodes usuelles ou transformer en sels par action d'un acide ou d'une base.

4. Variante du procédé de préparation selon la revendication 3 caractérisé en ce que l'on fait réagir le spiro 4',5'-dihydro /cyclohexane-1,6'/6H/-cyclopenta (b) thiophène/de formule (B):

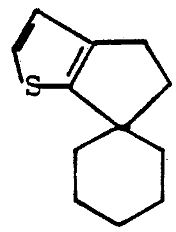

(B)

en présence d'un acide de Lewis, avec un dérivé bromé d'un acide de formule (II'):

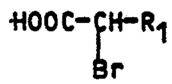

(II')

dans laquelle $R_1$ a la signification indiquée précédemment pour obtenir un produit de formule (V):

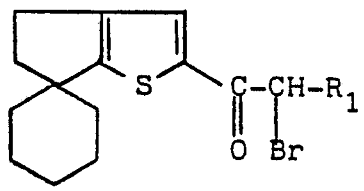

(V)

dans laquelle $R_1$ a la signification précédente, produit de formule (V) que l'on transforme en milieu acide par action d'un diol approprié en acétal cyclique de formule (VI):

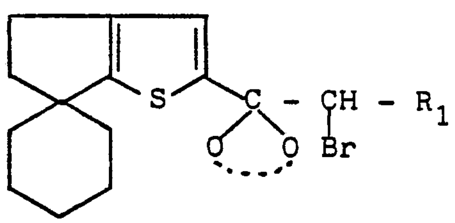

(VI)

dans laquelle $R_1$ a la signification précédente et le trait pointillé représente le reste du diol utilisé que l'on transforme par chauffage au sein d'un solvant polaire en ester de formule VII:

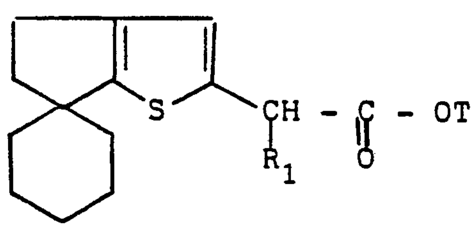

(VII)

dans laquelle $R_1$ a la signification précédente et T représente un reste alcoolique qui est fonction du diol et du solvant polaire utilisés, produit de formule (VII) que l'on saponifie pour obtenir le produit de formule (I) dans lequel Y représente le groupe $OR_2$ dans lequel $R_2$ est un atome d'hydrogène et $R_1$ a la signification indiquée précédemment et que le cas échéant l'on traite les produits de formule (I) ainsi obtenus comme indiqué à la revendication 3.

5. A titre de médicaments, les produits de formule (I) tels que définis à la revendication 1, sous leurs formes racémique ou optiquement actives, et leurs sels d'addition avec les acides ou les bases pharmaceutiquement acceptables.

6. A titre de médicaments, le produit de la revendication 2.

7. Les compositions pharmaceutiques contenant à titre de principe actif l'un au moins des médicaments tels que définis aux revendications 1 ou 2.

8. A titre de produits industriels nouveaux:

- les produits de formule (III) tels que définis à la revendication 3,

- les produits de formule (IV) tels que définis à la revendication 3,
- les produits de formule (V) tels que définis à la revendication 4,
- les produits de formule (VI) tels que définis à la revendication 4,
- le spiro 4',5'-dihydro /cyclohexane-1,6'/6H/-cyclopenta (b) thiophène,
- l'acide 4',5'-dihydro- α'-oxo spiro /cyclohexane 1,6'/6H/-cyclopenta (b) thiophène/2'-acétique.

**Patentansprüche**

1. Produkte der Formel (I):

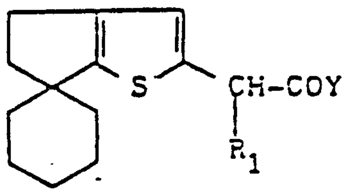

(I)

worin $R_1$ ein Wasserstoffatom oder einen Alkylrest mit 1 bis 5 Kohlenstoffatomen darstellt und Y entweder eine Gruppe $OR_2$ bedeutet, in der $R_2$ für ein Wasserstoffatom steht, oder einen Alkylrest mit 1 bis 5 Kohlenstoffatomen oder einen Rest

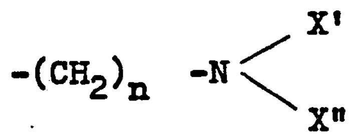

darstellt, worin n eine ganze Zahl von 2 bis 5 wiedergibt, X' und X", die gleich oder voneinander verschieden sind, ein Wasserstoffatom oder einen Alkylrest mit 1 bis 5 Kohlenstoffatomen bedeuten oder X' und X" gemeinsam mit dem Stickstoffatom, an das sie gebunden sind, einen gesättigten oder ungesättigten, heterocyclischen Rest mit 5 oder 6 Kettengliedern bilden, oder Y eine Gruppe

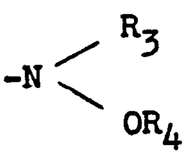

bedeutet, in der $R_3$ und $R_4$ gleich oder voneinander verschieden sind und für ein Wasserstoffatom oder einen Alkylrest mit 1 bis 5 Kohlenstoffatomen stehen, in ihren racemischen oder optisch aktiven Formen sowie deren Additionssalze mit Basen und Säuren.

2. 4',5'-Dihydro-α'-methyl-spiro-[cyclohexan-1,6,-[6H]-cyclopenta(b)-thiophen]-2'-essigsäure in racemischer oder optisch aktiver Form sowie deren Additionssalze mit Basen.

3. Verfahren zur Herstellung von Produkten der Formel (I), dadurch gekennzeichnet, daß man Spiro-[cyclohexan-1,6'-[6H]-cyclopenta(b)-thiophen]-4'-(5'-H)-on der Formel (A)

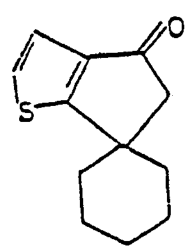

(A)

reduziert, um Spiro-4',5'-dihydro-[cyclohexan-1,6'-[6H]-cyclopenta(b)-thiophen] der Formel (B)

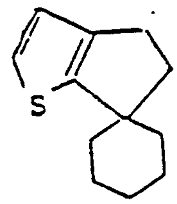

(B)

zu erhalten, welches man in Anwesenheit einer Lewissäure mit einem Derivat einer Säure der Formel (II)

HOOC-COOR' (II)

worin R' einen Alkylrest mit 1 bis 5 Kohlenstoffatomen darstellt, umsetzt, um ein Produkt der Formel (III)

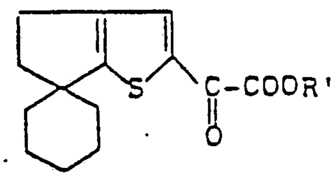

(III)

zu erhalten, das man verseift, um die 4',5'-Dihydro-α'-oxo-spiro-[cyclohexan-1,6'-[6H]-cyclopenta(b)-thiophen]-2'-essigsäure zu erhalten, und daß man

- <u>entweder</u> das Keton der vorstehenden Säure zum Methylen reduziert, um ein Produkt der Formel (I) zu erhalten, worin $R_1$ und $R_2$ ein Wasserstoffatom darstellen;
- <u>oder</u> die vorstehende Säure mit einem Organomagnesiumhalogenid der Formel $XMgR_1$, worin X für ein Halogenatom steht und $R_1$ einen Alkylrest mit 1 bis 5 Kohlenstoffatomen darstellt, behandelt, um ein Salz der Magnesiumverbindung der Formel (IV)

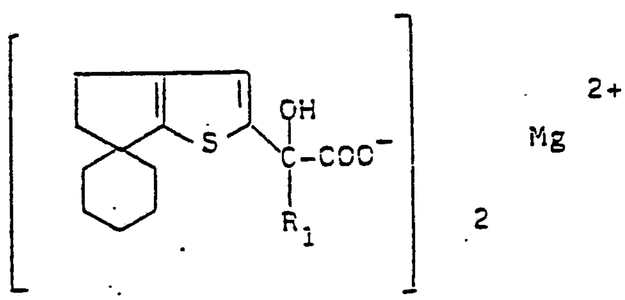

(IV)

zu erhalten, die man reduziert, um ein Produkt der Formel (I) zu erhalten, worin $R_1$ die vorstehend angegebene Bedeutung besitzt und Y die Gruppe $OR_2$ wiedergibt, in der $R_2$ für ein Wasserstoffatom steht, und daß man gewünschtenfalls die so erhaltenen Produkte der Formel (I), worin $R_2$ für ein Wasserstoffatom steht, zu einem Produkt der Formel (I) verestert, worin Y die Gruppe $OR_2$ darstellt, in der $R_2$ einen Alkylrest mit 1 bis 5 Kohlenstoffatomen oder einen Rest der Formel

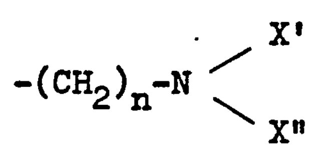

wiedergibt,

und daß man gegebenenfalls die so erhaltenen Produkte der Formel (I) mit einem Hydroxylamin der Formel

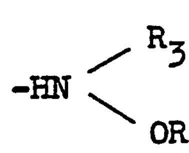

worin $R_3$ und $R_4$ die vorstehend angegebenen Bedeutungen besitzen, behandelt, um die Produkte der Formel (I) zu erhalten, worin Y eine Gruppe

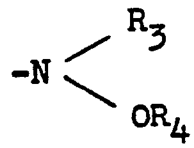

darstellt, welche Produkte der Formel (I) man gegebenenfalls nach üblichen Methoden aufspalten oder in Salze durch Einwirkenlassen einer Säure oder einer Base überführen kann.

4. Abänderung des Herstellungsverfahrens gemäß Anspruch 3, dadurch gekennzeichnet, daß man Spiro-4',5'-dihydro-[cyclohexan-1,6'-[6H]-cyclopenta(b)-thiophen] der Formel (B)

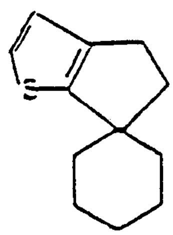

(B)

in Anwesenheit einer Lewissäure mit einem Bromderivat einer Säure der Formel (II')

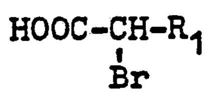

(II')

worin $R_1$ die vorstehend angegebene Bedeutung besitzt, umsetzt, um ein Produkt der Formel (V)

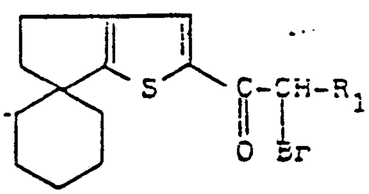

(V)

zu erhalten, worin $R_1$ die angegebene Bedeutung besitzt, das Produkt der Formel (V) in saurem Milieu durch Einwirkenlassen eines geeigneten Diols in ein cyclisches Acetal der Formel (VI)

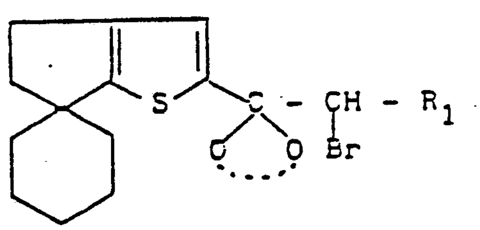

(VI)

überführt, worin $R_1$ die vorstehende Bedeutung besitzt und die gestrichelte Linie den Rest des verwendeten Diols anzeigt, welches man durch Erhitzen in dem Medium eines polaren Lösungsmittels in einen Ester der Formel VII

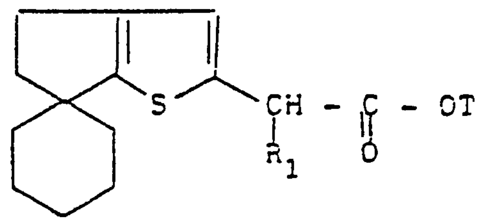

(VII)

überführt, worin $R_1$ die vorstehende Bedeutung besitzt und T einen alkoholischen Rest darstellt, der von dem verwendeten Diol und dem verwendeten polaren Lösungsmittel abhängt, das Produkt der Formel (VII) verseift, um das Produkt der Formel (I) zu erhalten, worin Y die Gruppe $OR_2$ wiedergibt, in der $R_2$ ein Wasserstoffatom bedeutet, und $R_1$ die vorstehend angegebene Bedeutung besitzt, und daß man gegebenenfalls die so erhaltenen Produkte der Formel (I), wie in Anspruch 3 angegeben, behandelt.

5. Als Arzneimittel die Produkte der Formel (I) gemäß Anspruch 1 in ihren racemischen oder optisch aktiven Formen und deren Additionssalze mit pharmazeutisch verträglichen Säuren und Basen.

6. Als Arzneimittel das Produkt gemäß Anspruch 2.

7. Pharmazeutische Zusammensetzungen, enthaltend als Wirkstoff zumindest eines der Arzneimittel gemäß den Ansprüchen 1 oder 2.

8. Als neue, industrielle Produkte
- die Produkte der Formel (III) gemäß Anspruch 3,
- die Produkte der Formel (IV) gemäß Anspruch 3,
- die Produkte der Formel (V) gemäß Anspruch 4,
- die Produkte der Formel (VI) gemäß Anspruch 4,
- Spiro-4',5'-dihydro-[cyclohexan-1,6'-[6H]-cyclopenta(b)-thiophen],
- 4',5'-Dihydro-α'-oxo-spiro-[cyclohexan-1,6'-[6H]-cyclopenta(b)-thiophen]-2'-essigsäure.

## Claims

1. Products of formula (I):

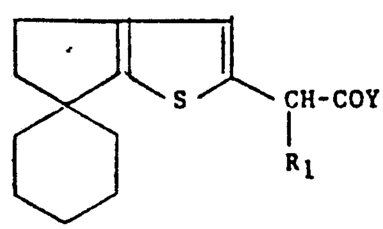

(I)

in which $R_1$ is a hydrogen atom or an alkyl radical containing from 1 to 5 carbon atoms and Y represents either an $OR_2$ group in which $R_2$ is a hydrogen atom or an alkyl radical containing from 1 to 5 carbon atoms or a radical:

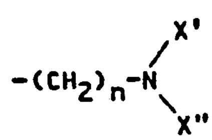

n being a whole number which can vary from 2 to 5, X' and X", identical or different, represent a hydrogen atom or an alkyl radical containing from 1 to 5 carbon atoms or X' and X" form, together with the nitrogen atom to which they are linked, a saturated or unsaturated heterocyclic radical, containing 5 or 6 links, or Y represents a group:

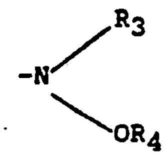

in which $R_3$ and $R_4$, identical or different, represent a hydrogen atom or an alkyl radical containing from 1 to 5 carbon atoms, in their racemic or optically active forms, as well as their addition salts with bases and acids.

2. 4',5'-dihydro-alpha'-methyl-spiro[cyclohexane-1,6'-[6H]-cyclopenta(b)thiophene]2'-acetic acid in racemic or optically active form as well as its addition salts with bases.

3. Process for the preparation of products of formula (I) characterized in that spiro[cyclohexane-1,6'-[6H]-cyclopenta(b)thiophene]-4'-(5'-H)-one of formula (A):

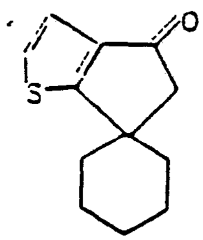

(A)

is reduced in order to obtain spiro 4',5'-dihydro[cyclohexane-1,6'-[6H]-cyclopenta(b)thiophene] of formula (B):

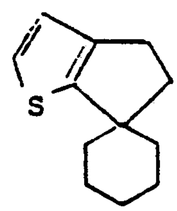

(B)

which is made to react in the presence of a Lewis acid, with a derivative of an acid of formula (II):

HOOC-COOR' (II)

R' being an alkyl radical containing from 1 to 5 carbon atoms, in order to obtain a product of formula (III):

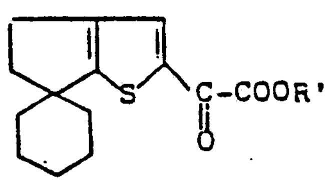

(III)

which is saponified in order to obtain 4',5'-dihydro-alpha-oxo-spiro[cyclohexane1,6'-[6H]-cyclopenta(b)thiophene]-2'-acetic acid and

- <u>either</u> the ketone of the acid previously mentioned is reduced to methylene in order to obtain a product of formula (I) in which $R_1$ and $R_2$ represent a hydrogen atom;
- <u>or</u> the acid previously mentioned is treated with an organomagnesium halide of formula $XMgR_1$, X being a halogen atom and $R_1$ an alkyl radical containing from 1 to 5 carbon atoms, in order to obtain a magnesium salt of formula (IV):

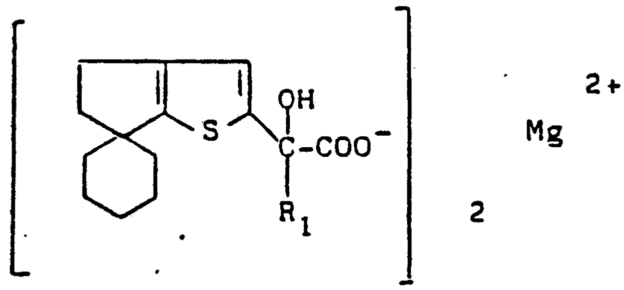

(IV)

which is reduced in order to obtain a product of formula (I) in which $R_1$ has the significance given previously and Y represents the group $OR_2$ in which $R_2$ is a hydrogen atom and if desired, the products of formula (I) thus obtained in which $R_2$ is a hydrogen atom are esterified into a product of formula (I) in which Y represents the group $OR_2$ in which $R_2$ is an alkyl radical containing from 1 to 5 carbon atoms or a radical:

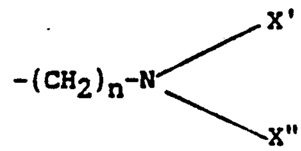

and if necessary or desired the products of formula (I) thus obtained are treated by a hydroxylamine with the formula:

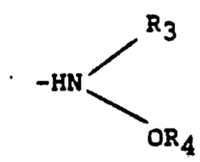

in which $R_3$ and $R_4$ have the significances indicated previously in order to obtain the products of formula (I) in which Y represents a group:

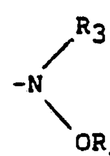

which products of formula (I) can, if necessary or desired, be resolved by the usual methods or converted into salts by the action of an acid or a base.

4. Variant of the preparation process according to claim 3 characterized in that spiro 4',5'-dihydro-[cyclohexane-1,6'-[6H]-cyclopenta(b)thiophene] of formula (B):

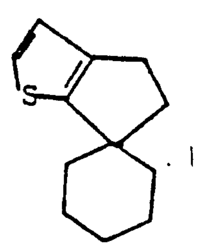

(B)

is made to react, in the presence of a Lewis acid, with a bromide derivative of an acid of formula (II'):

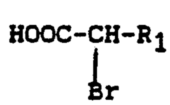

(II')

in which $R_1$ has the significance indicated previously, in order to obtain a product of formula (V):

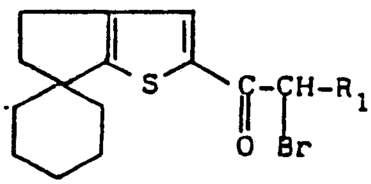

(V)

in which $R_1$ has the previous significance, which product of formula (V) is converted in acid medium by the action of an appropriate diol into cyclic acetal of formula (VI):

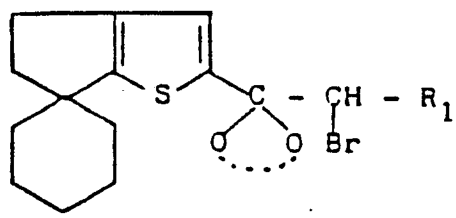

(VI)

in which $R_1$ has the previous significance and the broken line represents the residue of the diol used which is converted by heating in a polar solvent into an ester of formula (VII):

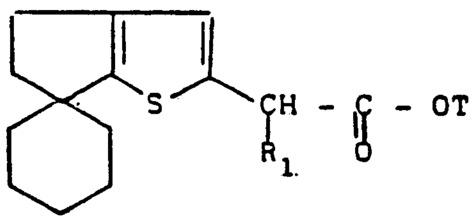

(VII)

in which $R_1$ has the previous significance and T represents an alcohol residue which is a function of the diol and the polar solvent used, which product of formula (VII) is saponified in order to obtain the product of formula (I) in which Y represents the group $OR_2$ in which $R_2$ is a hydrogen atom and $R_1$ has the significance indicated previously, and if necessary or desired, the products of formula (I) thus obtained are treated as indicated in claim 3.

5. As medicaments, the products of formula (I) as defined in claim 1, in their racemic or optically active forms, and their addition salts with pharmaceutically acceptable acids and bases.

6. As medicaments, the product of claim 2.

7. Pharmaceutical compositions containing as active principle at least one of the medicaments as defined in claims 1 or 2.

8. As new industrial products:
- the products of formula (III) as defined in claim 3,
- products of formula (IV) as defined in claim 3,
- products formula (V) as defined in claim 4,
- products of formula (VI) as defined in claim 4,
- spiro 4',5'-dihydro-[cyclohexane-1,6'-[6H]-cyclopenta(b)thiophene,
- 4',5'-dihydro-alpha'-oxo-spiro[cyclohexane-1,6'-[6H]-cyclopenta(b)thiophene]-2'-acetic acid.